# EUROPEAN PATENT APPLICATION

(11) **EP 3 760 709 A1**
(43) Date of publication of application: **06.01.2021**
(21) Application number: 19760781.5
(22) Date of filing: 28.02.2019
(51) Int. Cl.: C12N 5/074, C12N 5/10

(54) **CULTURE SYSTEM FOR CHEMICALLY INDUCING GENERATION OF PLURIPOTENT STEM CELLS AND CHEMICAL REPROGRAMMING METHOD USING SAME**

(30) Priority: 01.03.2018 CN 201810170553
(71) Applicant: Guangzhou Institutes Of Biomedicine And Health Chinese Academy Of Sciences, Guangzhou, Guangdong 510530 (CN)
(72) Inventor: PEI, Duanqing, Guangzhou, Guangdong 510530 (CN); CAO, Shangtao, Guangzhou, Guangdong 510530 (CN); LIU, Jing, Guangzhou, Guangdong 510530 (CN); YU, Shengyong, Guangzhou, Guangdong 510530 (CN); CHEN, Jiekai, Guangzhou, Guangdong 510530 (CN); YE, Jing, Guangzhou, Guangdong 510530 (CN); LI, Dongwei, Guangzhou, Guangdong 510530 (CN)
(74) Representative: Haseltine Lake Kempner LLP
(86) International application number: PCT/CN2019/076433
(87) International publication number: WO 2019/165988

(57) **Abstract**

Disclosed herein is a culture system for chemical induction of pluripotent stem cells, comprising a basic culture medium and a composition for performing chemical induction of reprogramming process. The said composition comprises a thymine analogue, a cAMP activator, a TGF-β receptor inhibitor, a bone morphogenetic protein, a RA receptor activator, a GSK3 inhibitor and a basic fibroblast growth factor. And the said culture system is free of serum. By using the culture system as described herein, there is no need to frequently replate cells during culture, such that culturing process is simplified and loss of cells resulting from replating cells is reduced. As the culture system is free of serum, subsequent collection of pluripotent stem cells and molecular mechanism analysis are simplified, thereby facilitating establishment of no animal origin culture systems for induction of pluripotent stem cells.

SEQUENCE LISTING

<110>  GUANGZHOU INSTITUTES OF BIOMEDICINE AND HEALTH, CHINESE ACADEMY OF
SCIENCES

<120>  CULTURE SYSTEM FOR CHEMICALLY INDUCING GENERATION OF PLURIPOTENT STEM
CELLS AND CHEMICAL REPROGRAMMING METHOD USING SAME

<160>  36

<170>  SIPOSequenceListing 1.0

<210>  1
<211>  18
<212>  DNA
<213>  Oct4 promoter sense sequence (Oct4)

<400>  1
cattgagaac cgtgtgag                                                    18

<210>  2
<211>  18
<212>  DNA
<213>  Oct4 promoter anti-sense sequence (Oct4)

<400>  2
tgagtgatct gctgtagg                                                    18

<210>  3
<211>  20
<212>  DNA
<213>  Nanog promoter sense sequence (Nanog)

<400>  3
ctcaagtcct gaggctgaca                                                  20

<210>  4
<211>  20
<212>  DNA
<213>  Nanog promoter anti-sense sequence (Nanog)

<400>  4
tgaaacctgt ccttgagtgc                                                  20

<210>  5
<211>  20
<212>  DNA
<213>  Sox2 promoter sense sequence (Sox2)

<400>  5
agggctggga gaaagaagag                                                  20

<210>  6
<211>  20
<212>  DNA
<213>  Sox2promoter anti-sense sequence (Sox2)

<400>  6
ccgcgattgt tgtgattagt                                                  20

<210>  7
<211>  20
<212>  DNA

<213>  Esrrb promoter sense sequence (Esrrb)

<400>  7
tttctggaac ccatggagag                                                    20

<210>  8
<211>  20
<212>  DNA
<213>  Esrrb promoter anti-sense sequence (Esrrb)

<400>  8
agccagcacc tccttctaca                                                    20

<210>  9
<211>  18
<212>  DNA
<213>  Dppa5 promoter sense sequence (Dppa5)

<400>  9
ccgtgcgtgg tggataag                                                      18

<210>  10
<211>  19
<212>  DNA
<213>  Dppa5 promoter anti-sense sequence (Dppa5)

<400>  10
gcgactggac ctggaatac                                                     19

<210>  11
<211>  20
<212>  DNA
<213>  Rex1 promoter sense sequence (Rex1)

<400>  11
cagccagacc accatctgtc                                                    20

<210>  12
<211>  23
<212>  DNA
<213>  Rex1 promoter anti-sense sequence (Rex1)

<400>  12
gtctccgatt tgcatatctc ctg                                                23

<210>  13
<211>  19
<212>  DNA
<213>  Sall4 promoter sense sequence (Sall4)

<400>  13
ctaaggagga agaggagag                                                     19

<210>  14
<211>  18
<212>  DNA
<213>  Sall4 promoter anti-sense sequence (Sall4)

<400>  14
caaggctatg gtcacaag                                                      18

<210>  15
<211>  20

<212>  DNA
<213>  Sox17 promoter sense sequence (Sox17)

<400>  15
cagtatctgc cctttgtgta                                                    20

<210>  16
<211>  19
<212>  DNA
<213>  Sox17 promoter anti-sense sequence (Sox17)

<400>  16
gcaatagtag accgctgag                                                     19

<210>  17
<211>  19
<212>  DNA
<213>  Foxa2 promoter sense sequence (Foxa2)

<400>  17
gcagacactt cctactacc                                                     19

<210>  18
<211>  18
<212>  DNA
<213>  Foxa2 promoter anti-sense sequence (Foxa2)

<400>  18
tccactcagc ctctcatt                                                      18

<210>  19
<211>  18
<212>  DNA
<213>  Gata4 promoter sense sequence (Gata4)

<400>  19
cagcagcagt gaagagat                                                      18

<210>  20
<211>  19
<212>  DNA
<213>  Gata4 promoter anti-sense sequence (Gata4)

<400>  20
gtctgagtga caggagatg                                                     19

<210>  21
<211>  23
<212>  DNA
<213>  Gata6 promoter sense sequence (Gata6)

<400>  21
ggtctctaca gcaagatgaa tgg                                                23

<210>  22
<211>  19
<212>  DNA
<213>  Gata6 promoter anti-sense sequence (Gata6)

<400>  22
tggcacagga cagtccaag                                                     19

<210>  23

<211>  21
<212>  DNA
<213>  Cdh1 promoter sense sequence (Cdh1)

<400>  23
cagccttctt ttcggaagac t                                                21

<210>  24
<211>  22
<212>  DNA
<213>  Cdh1 promoter anti-sense sequence (Cdh1)

<400>  24
ggtagacagc tccctatgac tg                                               22

<210>  25
<211>  20
<212>  DNA
<213>  Epcam promoter sense sequence (Epcam)

<400>  25
cttgtgtctg cacgacctgt                                                  20

<210>  26
<211>  22
<212>  DNA
<213>  Epcam promoter anti-sense sequence (Epcam)

<400>  26
ccaagcattt agacgccagt tt                                               22

<210>  27
<211>  19
<212>  DNA
<213>  Cdh2 promoter sense sequence (Cdh2)

<400>  27
ccatcatcgc tatccttct                                                   19

<210>  28
<211>  20
<212>  DNA
<213>  Cdh2 promoter anti-sense sequence (Cdh2)

<400>  28
cctccacctt cttcatcata                                                  20

<210>  29
<211>  23
<212>  DNA
<213>  CD44 promoter sense sequence (CD44)

<400>  29
cgttaatgtt gatggctcct tac                                              23

<210>  30
<211>  18
<212>  DNA
<213>  CD44 promoter anti-sense sequence (CD44)

<400>  30
gtcctggttc gcacttga                                                    18

<210>  31
<211>  20
<212>  DNA
<213>  Twist2 promoter sense sequence (Twist2)

<400>  31
agatgaccag ctgcagctac                                                    20

<210>  32
<211>  18
<212>  DNA
<213>  Twist2 promoter anti-sense sequence (Twist2)

<400>  32
atgtgcaggt gggtcctg                                                      18

<210>  33
<211>  20
<212>  DNA
<213>  Snail promoter sense sequence (Snail)

<400>  33
ctcggatgtg aagagatacc                                                    20

<210>  34
<211>  18
<212>  DNA
<213>  Snail promoter anti-sense sequence (Snail)

<400>  34
agactcttgg tgcttgtg                                                      18

<210>  35
<211>  25
<212>  DNA
<213>  Gapdh promoter sense sequence (Gapdh)

<400>  35
aactttggca ttgtggaagg gctca                                              25

<210>  36
<211>  25
<212>  DNA
<213>  Gapdh promoter anti-sense sequence (Gapdh)

<400>  36
ttggcagcac cagtggatgc aggga                                              25

## Description

### CROSS REFERENCE TO RELATED APPLICATIONS

This application claims priority to Chinese Patent Application Serial No. 201810170553.X, filed on March 1, 2018, entitled "CULTURE SYSTEM FOR CHEMICALLY INDUCING GENERATION OF PLURIPOTENT STEM CELLS AND CHEMICAL REPROGRAMMING METHOD USING SAME", the entire disclosures of which are herein incorporated by reference.

### FIELD OF THE INVENTION

The present invention relates to a culture system for chemical induction of pluripotent stem cells and a method for performing chemical induction of reprogramming process by using the said culture system.

### BACKGROUND OF THE INVENTION

Induction of pluripotent stem cells or iPSCs from somatic cells is a revolutionary concept for biology and medicine. For the latter, it empowers regenerative medicine as patient specific stem cells, which can be applied for treatment development against degenerative conditions such as Parkinson's and Alzheimer's diseases. For the former, the iPSCs reprogramming process driven by the Yamanaka factors Oct4, Sox2, Klf4 and Myc has been analyzed in great details, providing insights into the molecular and cellular mechanisms governing cell fate and its transitions. The detailed understanding of cell fate has also impacted therapeutic development in areas such as cancer, neurological disorders, cardiovascular diseases.

It has been proposed that the Yamanaka factors may be replaced by chemical molecules. For example, Deng and his colleagues reported the replacement of Oct4 with small molecule Forskolin and ciPSC generation (Hou, P. et al., Pluripotent Stem Cells Induced From Mouse Somatic Cells by Small-molecule Compounds, Science 341, 651-654). However, the reported culture system with chemical molecules remains inefficient and require replating cells frequently. There is a need to improve the culture systems with chemical molecules (Zhao Y. et al. , A XEN-like State Bridges Somatic Cells to Pluripotency During Chemical Reprogramming. Cell, 163, 1678-1691).

Therefore, it still needs more effort to replace Yamanaka factors with chemical small molecules for chemical induction of pluripotent stem cells.

### SUMMARY OF THE INVENTION

In one aspect of the present invention, provided herein is a culture system for chemical induction of pluripotent stem cells, which comprises a culture medium and a group consisting of: a thymine analogue, a cAMP activator, a TGF-β receptor inhibitor, a bone morphogenetic protein, a RA receptor activator, a GSK3 inhibitor and a basic fibroblast growth factor, wherein the said culture system is free of serum.

In one embodiment of the present invention, the said culture system can further comprises vitamin C, DOT1L inhibitor, histone deacetylase inhibitor and/or DZNep.

In one embodiment, the said culture system further comprises EZH2 inhibitor and/or cMet inhibitor.

In one embodiment, the thymine analogue has a concentration from 0.01µM to 10 µM. In one embodiment, the cAMP activator has a concentration from 0.01 µM to 10 µM. In one embodiment, the TGF-β receptor inhibitor has a concentration from 0.01 µM to 5 µM. In one embodiment, the bone morphogenetic protein has a concentration from 0.01 ng/ml to 10 ng/ml. In one embodiment, the basic fibroblast growth factor has a concentration from 0.01 ng/ml to 10 ng/ml. In one embodiment, the RA receptor activator has a concentration from 0.01 µM to 0.05 µM. In one embodiment, the GSK3 inhibitor has a concentration from 0.01 µM to 3 µM. In one embodiment, the thymine analogue is Brdu. In one embodiment, the cAMP activator is FSK. In one embodiment, the TGF-β receptor inhibitor is RepSox. In one embodiment, the bone morphogenetic protein is BMP4. In one embodiment, the basic fibroblast growth factor is FGF2. In one embodiment, the RA receptor activator is AM580. In one embodiment, the GSK3 inhibitor is CHIR99021. In one embodiment, the vitamin C has a concentration from 0.01 µg/ml to 50 µg/ml. In one embodiment, the DOT1L inhibitor has a concentration from 0.01 µM to 5 µM. In one embodiment, the histone deacetylase inhibitor has a concentration from 0.01 mM to 0.5 mM. In one embodiment, the DZNep has a concentration from 0.001 µM to 0.05 µM. In one embodiment, the DOT1L inhibitor is EPZ5676 and/or SGC0946. The histone deacetylase inhibitor is valproic acid. In one embodiment, the EZH2 inhibitor has a concentration from 0.01 µM to 10 µM. In one embodiment, the cMet inhibitor has a concentration from 0.01 µM to 10 µM. In one embodiment, the EZH2 inhibitor is EPZ6438. In one embodiment, cMet inhibitor is Capmatinib.

In one illustrative embodiment, the culture system for chemical induction of pluripotent stem cells comprises a culture medium such as iCD1 and the following ingredients:
10ng/ml BMP4,
50µg/ml vatimin C,
5µM EPZ5676,
10µM Brdu,
0.05µM AM580,
3 µM CHIR99021,
10ng/ml FGF2,
10µM Forsklin,
5µM RepSox,
0.1 mM valproic acid,
0.05µM DZNep, and/or
5µM SGC0946.

In another illustrative embodiment, the culture system for chemical induction of pluripotent stem cells comprises a culture medium such as iCD1 and the following ingredients:
10ng/ml BMP4,
50µg/ml vatimin C,
5µM EPZ5676,
10µM Brdu,
0.05µM AM580,
3 µM CHIR99021,
10ng/ml FGF2,
10µM Forsklin,
5µM RepSox,
0.1 mM valproic acid,
0.05µM DZNep,
5µM SGC0946,
5 µM EPZ643 8, and/or
5µM Capmatinib.

In another aspect of the present invention, provided herein is a culture system for chemical induction of pluripotent stem cells, comprising a basic culture medium and a composition for performing chemical induction of reprogramming process. The said composition comprises a thymine analogue, a cAMP activator, a TGF-β receptor inhibitor, a bone morphogenetic protein, a RA receptor activator, a GSK3 inhibitor and a basic fibroblast growth factor. And the said culture system is free of serum.

In one embodiment, the thymine analogue has a concentration from 0.01µM to 10 µM. In one embodiment, the cAMP activator has a concentration from 0.01 µM to 10 µM. In one embodiment, the TGF-β receptor inhibitor has a concentration from 0.01 µM to 5 µM. In one embodiment, the bone morphogenetic protein has a concentration from 0.01 ng/ml to 10 ng/ml. In one embodiment, the basic fibroblast growth factor has a concentration from 0.01 ng/ml to 10 ng/ml. In one embodiment, the RA receptor activator has a concentration from 0.01 µM to 0.05 µM. In one embodiment, the GSK3 inhibitor has a concentration from 0.01 µM to 3 µM. In one embodiment, the thymine analogue is Brdu. In one embodiment, the cAMP activator is FSK. In one embodiment, the TGF-β receptor inhibitor is RepSox. In one embodiment, the bone morphogenetic protein is BMP4. In one embodiment, the basic fibroblast growth factor is FGF2. In one embodiment, the RA receptor activator is AM580. In one embodiment, the GSK3 inhibitor is CHIR99021.

In one embodiment, the said composition can further comprises vitamin C, DOT1L inhibitor, histone deacetylase inhibitor and/or DZNep.

In one embodiment, the vitamin C has a concentration from 0.01 µg/ml to 50 µg/ml. In one embodiment, the DOT1L inhibitor has a concentration from 0.01 µM to 5 µM. In one embodiment, the histone deacetylase inhibitor has a concentration from 0.01 mM to 0.5 mM. In one embodiment, the DZNep has a concentration from 0.001 µM to 0.05 µM.

In one embodiment, the DOT1L inhibitor is EPZ5676 and/or SGC0946. The histone deacetylase inhibitor is valproic acid.

In one embodiment, the said composition can further comprises EZH2 inhibitor and/or cMet inhibitor. In one embodiment, the EZH2 inhibitor has a concentration from 0.01 µM to 10 µM. In one embodiment, the cMet inhibitor has a concentration from 0.01 µM to 10 µM. In one embodiment, the EZH2 inhibitor is EPZ6438. In one embodiment, cMet inhibitor is Capmatinib.

In one illustrative embodiment, the culture system for chemical induction of pluripotent stem cells comprises a basic culture medium such as iCD1 and the composition for performing chemical induction of reprogramming process comprising:
10ng/ml BMP4,
50µg/ml vatimin C,
5µM EPZ5676,
10µM Brdu,
0.05µM AM580,
3 µM CHIR99021,
10ng/ml FGF2,
10µM Forsklin,
5µM RepSox,
0.1 mM valproic acid,
0.05µM DZNep, and/or
5µM SGC0946.

In another illustrative embodiment, the culture system for chemical induction of pluripotent stem cells comprises a basic culture medium such as iCD1 and the composition for performing chemical induction of reprogramming process comprising:
10ng/ml BMP4,
50µg/ml vatimin C,
5µM EPZ5676,
10µM Brdu,
0.05µM AM580,
3 µM CHIR99021,
10ng/ml FGF2,
10µM Forsklin,
5µM RepSox,
0.1 mM valproic acid,
0.05µM DZNep,
5µM SGC0946,
5µM EPZ643 8, and/or
5µM Capmatinib.

In a further aspect of the present invention, provided herein is a method for chemically inducing somatic cells to perform reprogramming process, comprising culturing the somatic cells in the culture system as described herein for a time period that is sufficient to reprogram the somatic cells to pluripotent state. The method further comprises changing the culture medium to DEME medium supplement with PD0325901, non-essential amino acids, GlutaMax™, human white cell antigen B27, CHIR99021, leukaemia inhibitory factor, and/or N2 additive after the somatic cells have been reprogrammed to pluripotent state and culturing the reprogrammed cells in the said DEME medium for a second time period that is sufficient to maintain the native state of the embryonic stem cells. In one embodiment, PD0325901 has a concentration of 1 µM. In one embodiment, the non-essential amino acids are comprised of 1% of the total volume of the medium. In one embodiment, GlutaMax™ is comprised of 1% of the total volume of the medium. In one embodiment, the human white cell antigen B27 is comprised of 2% of the total volume of the medium. In one embodiment, CHIR99021 has a concentration of 3 µM. In one embodiment, the N2 additive is comprised of 1% of the total volume of the medium. In one embodiment, the leukaemia inhibitory factor has a concentration from 1U to 1000U.

The time period that is sufficient to reprogram the somatic cells to pluripotent state can be determined by observing the changes in cell morphology and formation of clones during culture. In one embodiment, the time period that is sufficient to reprogram the somatic cells to pluripotent state can be 8 days to 22 days. The second time period that is sufficient to maintain the native state of the embryonic stem cells can be determined by observing the changes in cell morphology and formation of clones during culture. In one embodiment, the second time period that is sufficient to maintain the native state of the embryonic stem cells can be 1 day to 18 days.

The somatic cells that can be reprogrammed by the method as provided herein may include, but not limited to, fibroblasts, bone-marrow derived monocytes, skeletal muscle cells, adipocytes, peripheral blood monouclear cells, macrophages, hepatocytes, keratinocytes, oral keratinocytes, hair follicle dermal cells, stomach epithelial cells, lung epithelial cells, synoviocytes, renal cells, skin epithelial cells, osteoblasts, neural stem cells and dermal cells.

In yet another aspect of the present invention, provided herein is a kit for chemical induction of pluripotent stem cells, comprising the culture system as described herein.

During performing chemical induction of reprogramming process on the somatic cells by using the method as described herein, there is no need to frequencely replate cells. Therefore, the reprogramming process is simplified, and loss of cells resulting from replating cells is reduced. As the culture system as decribed herein is free of serum, subsequent collection of pluripotent stem cells and molecular mechanism analysis will be simplified, thereby facilitating establishment of no animal origin culture systems for induction of pluripotent stem cells.

### BRIEF DESCRIPTION OF THE DRAWINGS

Figure 1 depicts a two-stage method for chemical induction of somatic cells to perform reprogramming process according to one embodiment as described herein.
Figure 2 shows the expression levels of endogenous pluripotent genes Oct4, Nanog, Sox2, Esrb, Rex1Dappa5, Sall4 and Cdhl in mouse embryonic fibroblasts, chemically induced pluripotent stem cells and mouse embryonic stem cells. CiPS-1, CiPS-2 and CiPS-3 represent chemically induced pluripotent stem cells from different clones.
Figure 3 depicts transcriptomic profiles of chemically induced pluripotent stem cells, mouse embryonic stem cells and mouse fibroblasts.
Figure 4 shows the protein expression levels of pluripotent genes Oct4, Nanog, Sox2, Esrb and Rex1 in chemically induced pluripotent stem cells.
Figure 5 shows that the chemically induced pluripotent stem cells form teratoma in mouse and differentiate into three germ layers.
Figure 6 shows that the chemically induced pluripotent stem cells can be maintained with normal karyotype during passage.
Figure 7 shows that chimeric mice were obtained by injecting the chemically induced pluripotent stem cells into pseudopregnancy mice.

### DETAILED DESCRIPTION OF THE INVENTION

Unless otherwise defined, all technical and scientific terms used herein have the same meaning as commonly understood by one of ordinary skill in the art to which this invention belongs.

The terminology used herein is for the purpose of describing particular embodiments only and is not intended to be limiting of the invention. As used herein, the singular forms "a", "an" and "the" are intended to include the plural forms as well, unless the context clearly indicates otherwise.

### Definition

As used herein, the term "pluripotent" or "pluripotency" means that cells can differentiate into different kinds of cells that can be found in adult animals developed from the progeny thereof.

As used herein, the term "differentiate" refers to a process through which less specialized cells will become specialized cells so as to form progeny of at least one new type of cell. The term "undifferentiate" refers to a process in which partially differentiated cells or terminally differentiated cells are returned to prior development stages, such as pluripotency stage. The term "transdifferentiate" refers to a process that converts one differentiated cell type to another differentiated cell type. Under certain conditions, the progeny with profiles of new cell type can be comprised of at least about 1%, 5%, 25% or more.

As used herein, the term "somatic cells" are selected from mouse somatic cells and human somatic cells. Preferably, the mouse somatic cells and the human somatic cells are selected from the group consisting of fibroblasts, bone marrow derived mononuclear cells, skeletal muscle cells, adipocytes, peripheral blood mononuclear cells, macrophages, neural stem cells, hepatic cells, keratinocytes, oral keratinocytes, hair follicle dermal cells, gastric epithelial cells, lung epithelial cells, synovial cells, renal cells, skin epithelial cells, osteoblast cells, and dermal cells. As used herein, the term "isolated" refers to isolate cells mechanically or chemically. Examples of the isolated cells include developing cell mass, cell cultures and cell lines.

As used herein, the term "inhibitors" or "activators" associated with expression activity refer to inhibiting molecules or activating molecules identified by detecting expression or activity for target proteins (or encoded polynucleotides) *in vivo* or *in vitro,* for example, ligands, agonists, antagonists, and homologs and simulants thereof. The inhibitors refer to agents, for example, which inhibit expression or combination, partially or completely block activation or activity of protease inhibitors, reduce, prevent, delay activation, inactivation, destabilization, or down-regulate activity of target proteins, such as antagonists. The activators refer to agents, for example, which induce or activate expression or combination of the target proteins, simulate, increase, open, activate, facilitate, enhance activation or activity of protease inhibitors, sensitize or up-regulate activity of the target proteins (or encoded polynucleotides), such as agonists. Detection of inhibitors or activators includes, for example, applying specific regulators to cells expressing the target proteins and then determining effect on activity of the target proteins. Treatment by the candidate activators or inhibitors includes comparison of the target proteins with the control samples without treatment by inhibitors or activators, so as to detect the effect on the target proteins. Control samples (without treatment by regulators) have 100% relative activity value. When the activity value is about 80%, 50%, 25%, 10%, 5% or 1% relative to control, the target proteins are inhibited. When the activity value is about 110%, 150%, 200%, 300%, 400%, 500% or 1000%-3000% or higher relative to control, the target proteins are activated.

### Culture system for Chemical Induction of Pluriotent Stem Cells

In one aspect of this embodiment, provided herein is a culture system for chemical induction of pluripotent stem cells, comprising a basic medium and a composition for performing chemical induction of reprogramming process. The composition for performing chemical induction of reprogramming process comprises a thymine analogue, a cAMP activator, a TGF-β receptor inhibitor, a bone morphogenetic protein, a RA receptor activator, a GSK3 inhibitor and a basic fibroblast growth factor, and the said culture system is free of serum. The composition for performing chemical induction of reprogramming process may further comprise vitamin C, DOT1L inhibitor, histone deacetylase inhibitor and/or DZNep, and may further comprise EZH2 inhibitor and/or cMet inhibitor. Each ingredient in the culture system will be described in detail as below.

### I. Thymine Analogue

The thymine analogue as described herein refers to a chemical compound with similar structure to thymine, which is 5-bromodeoxyuridine (Brdu) with a feature that methyl connected with C on 5 position of thymine ring is substituted by bromine. The thymine analogue can compete with endogenous thymine to incorporate into newly synthesized DNA when synthesizing DNA by cell proliferation. Brdu has a molecular weight of 307.1 with a chemical structure as below:

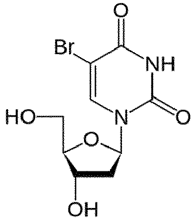

In the culture system for chemical induction of pluripotent stem cells as described herein, Brdu has a concentration from 0.01 to 10 µM, or 0.1 to 10 µM, or 1 to 10 µM, or 2 to 10 µM, or 3-10 µM, or 4 to 10 µM, or 5 to 10 µM, or 6 to 10 µM, or 7 to 10 µM, or 8 to 10 µM, or 9 to 10 µM, preferably, 3 µM, 5 µM, 8 µM or 10 µM.

### II. cAMP Activator

Adenylate cyclase (cAMP) is an integral membrane protein, which is able to convert ATP to cAMP so as to result in signal response to cells, and is an effctor in G protein conjugation system. The activator of the adenylate cyclase (cAMP) as described herein can activate the adenylate cyclase via cells, such that the cAMP level in cells can be increased. Illustrative cAMP activators include, but not limited to, FSK, FSH, milrinone, cilostamide, rolipram, dbcAMP, 8-Br-cAMP, IBMX, PGE2, NKH477, sp-8-br-cAMP. cAMP activator as used in the culture system as described herein is preferably FSK. In the culture system for chemical induction of pluripotent stem cells as described herein, FSK has a concentration from 0.01 to 10 µM, or 0.1 to 10 µM, or 1 to 10 µM, or 2 to 10 µM, or 3-10 µM, or 4 to 10 µM, or 5 to 10 µM, or 6 to 10 µM, or 7 to 10 µM, or 8 to 10 µM, or 9 to 10 µM, preferably, 3 µM, 5 µM, 8 µM or 10 µM.

### III. TGF-β Receptor Inhibitor

TGF-β receptor inhibitor as described herein is able to inhibit TGF-β signal pathway and activate cell specific function differentiation. Examples of TGF-β receptor inhibitor include, but not limited to, Repsox (E-616452), SB431542, A8301, GW788388, SD208, SB525334, LY364947, D4476, SB505124, GW6604, SU5416, CAT-152, CAT-192, SB431542, SD-208, SM16, NPC-30345, Ki26894, SB-203580, SD-093, Gleevec. In one embodiment, TGF-β receptor inhibitor used in the culture system as described herein is preferably RepSox. RepSox has a concentration from 0.01 to 5µM, or 0.1 to 5µM, or 1 to 5µM, or 2 to 5µM, or 3 to 5µM, or 4 to 5µM, preferably 1µM, 3µM, 5µM.

### IV. Bone Morphogenetic Protein (BMP)

BMP is a glycoprotein with low molecular weight (about 30,000 Da), free of collagen. A mature BMP molecule is a double-chain (each chain contains 400 amino acids) polypeptide dimer molecule fixed by disulfide bond of cysteine. BMP is synthesized in a form of a big precursor protein and comprises signal peptide moiety, front domain and carboxy terminal area, which is synthesized into a dimer by cutting carboxyl terminal from precursor protein with proteolytic enzyme. BMP4 as used herein is a critical osteoblast factor, which participate into early lesion of heterotopic ossification. BMP4 plays a role in stimulating expression of type I collagen, alkaline phosphatase and osteocalcin. In the culture system for chemical induction of pluripotent stem cells as described herein, BMP4 has a concentration from 0.01 to 10 ng/ml, or 0.1 to 10 ng/ml, or 1 to 10 ng/ml, or 2 to 10 ng/ml, or 3 to 10 ng/ml, or 4 to 10 ng/ml, or 5 to 10 ng/ml, or 6 to 10 ng/ml, or 7 to 10 ng/ml, or 8 to 10 ng/ml, or 9 to 10 ng/ml, preferably 1 ng/ml, 3 ng/ml, 5 ng/ml, 8 ng/ml or 10 ng/ml.

### V. RA Receptor Activator

Retinoic acids play an important role in regulation of cell growth, differentiation, apoptosis and the like. Their metabolic products with physiological activity include all-trans retinoic acids, 13-cis-RA and 9-cis-RA, which can combine to DNA response element via RA receptor to regulate transcription of target genes. Illustrative RA receptor activators include, but not limited to, TTNPB, Ch55, Retinol, AM580, ATRA, 13-cis-RA, and Retinoic. In the culture system for chemical induction of pluripotent stem cells as described herein, RA receptor activator is preferably AM580, which has a concentration from 0.01 to 0.05 µM, or 0.02 to 0.05 µM, or 0.03 to 0.05 µM, or 0.04 to 0.05 µM, preferably 0.01 µM, 0.03 µM or 0.05 µM.

### V. GSK3 Inhibitor

Glycogen synthase kinase-3 (GSK-3) generally exists in eukaryocytes of mammals, which has effect on various signal proteins, structural proteins and transcription factors to regulate cell differentiation, proliferation, survival and apoptosis, in addition to regulation of activity of glycogen synthase. For example, GSK3 inhibitors can activate Wnt/β-catenin pathway. A lot of β-catenin downstream genes co-regulate pluripotent gene network. For example, GSK inhibitors can activate cMyc expression and enhance protein stability and transcription activity thereof. Examples of GSK3 inhibitors include, but not limited to, CHIR99021, CHIR98014, TD114-2, BIO, Kenpaullone, TWS119, CBM1078, SB216763, 3F8(TOCRIS), AR-A014418, FRATide, Indirubin-3'-monoxime, L803, CT99021, CT20026, SB415286. GSK3 inhibitor used in the culture system as described herein is preferably CHIR99021, which has a concentration from 0.01 to 3 µM, or 0.1 to 3 µM, or 0.5 to 3 µM, or 1 to 3 µM, or 1.5 to 3 µM, or 2 to 3 µM, or 2.5 to 3 µM, preferably 0.5 µM, 1 µM, 2 µM, or 3 µM.

### VII. Basic Fibroblast Growth Factor

Basic fibroblast growth factor is a polypeptide being capable of facilitating differentiation of mesoderm cells and neural ectoderm cells with strong angiogenesis effect. *In vitro,* the basic fibroblast growth factor is able to stimulate cell proliferation and migration, and induce plasminogen activator and collagenase activity, which is a mitogen with high affinity to heparin. The basic fibroblast growth factor used in the culture system as described herein has a concentration from 0.01 to 10ng/mL, or 0.1 to 10ng/mL, or 0.5 to 10ng/mL, or 1 to 10ng/mL, or 2 to 10ng/mL, or 3 to 10ng/mL, or 4 to 10ng/mL, or 5 to 10ng/mL, or 6 to 10ng/mL, or 7 to 10ng/mL, or 8 to 10ng/mL, or 9 to 10ng/mL, preferably 1ng/ml, 3ng/ml, 5ng/mL, 8ng/ml, or 10ng/mL.

### VIII. DOT1L Inhibitor

Proteins encoded by DOT1L genes are a histone methyltransferase for methylating lysine-79 of histone H3, which is inactive against free core histone and exhibits significant histone methyltransferase activity against nucleosome. DOT1L inhibitor used herein is EPZ5676 and SGC0946. EPZ5676 used in the culture system as described herein has a concentration from 0.01 to 5µM, or 0.1 to 5µM, 0.5 to 5 µM, or 1 to 5µM, or 2 to 5µM, or 3 to 5µM, or 4 to 5µM, preferably 0.5 µM, µM, 3µM, 5µM. SGC0946 used in the culture system as described herein has a concentration from 0.01 to 5µM, or 0.1 to 5µM, 0.5 to 5 µM, or 1 to 5µM, or 2 to 5µM, or 3 to 5µM, or 4 to 5µM, preferably 0.5 µM, µM, 3µM, 5µM.

### IX. Histone Deacetylase Inhibitor

Histone deacetylase inhibitor is a chemical compound with a function of interference histone deacetylase. Histone deacetylase inhibitor can be generally divided into NAD+dependent enzyme and Zn2+ dependent enzyme. Zn2+dependent enzyme includes subgroups I, II, IV of HDAC and NAD+dependent enzyme is subgroup III of HDAC. Histone deacetylase inhibitors inhibit proliferation of tumor cells and induce cell differentiation and (or) apoptosis through increasing acetylation degree of histone in cells and enhancing expression level of gene such as p21. The histone deacetylase inhibitor used in the culture system as described herein is valproic acid (VPA) pertaining to HDAC subgroup, which has a concentration from 0.01mM to 0.5mM, or 0.05 to 0.5mM, or 0.1 to 0.5mM, or 0.2 to 0.5mM, or 0.3 to 0.5mM, or 0.4 to 0.5mM, preferably 0.1mM, 0.3mM or 0.5mM.

### X. DZNep

3-Deazaneplanocin A (DZNep) is an adenosine analogue, which is a competitive inhibitor for S-adenosylhomocysteine hydrolase and has a chemical structure as below:

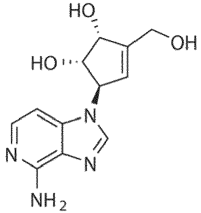

In the culture system for chemical induction of pluripotent stem cells as described herein, DZNep has a concentration from 0.001 to 0.05 µM, or 0.005 to 0.05 µM, or 0.01 to 0.05 µM, or 0.02 to 0.05 µM, or 0.03 to 0.05 µM, or 0.04 to 0.05 µM, preferably 0.01 µM, 0.03 µM, or 0.05 µM.

### XI. EZH2 Inhibitor

EZH2 is an intracellular histone methyltransferase, which is able to facilitate methylation of trimethyl on the 27^{th} amino acid of intracellular histone H3 (H3K27me3) and is proven to be a protooncogene and associated with growth and metastasis of a variety of tumors. EZH2 can inhibit expression of cancer suppressor genes by enhancing methylation level of H3K27me3 in cells, thereby resulting in tumorigenesis. EZH2 inhibitor can be a small molecule compound, including, but not limited to, GSK503, GSK343, EPZ005687, and EPZ6438 with respective structures as below.

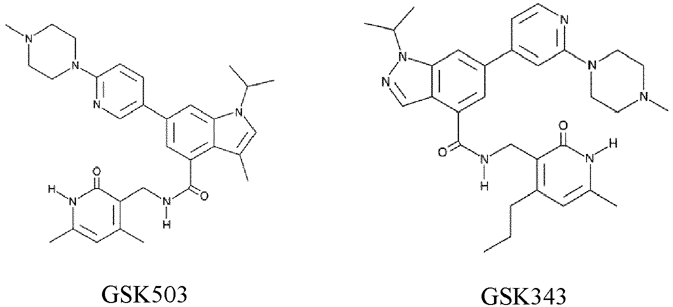

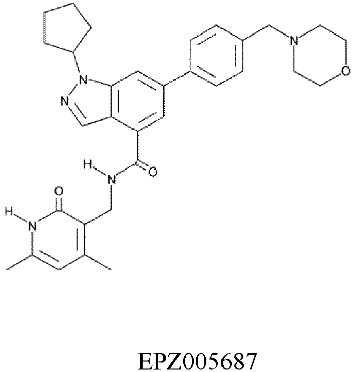

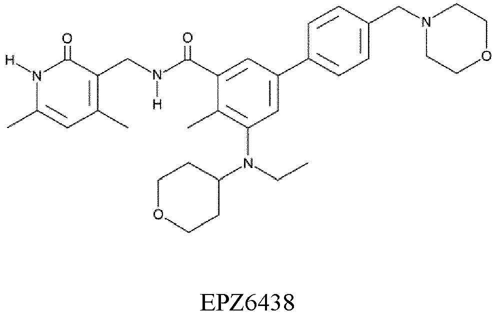

EZH2 inhibitor used in the culture system as described herein is preferably EPZ6438 with the structure as shown above, which has a concentration from 0.01 to 10 µM, or 0.1 to 10 µM, or 1 to 10 µM, or 2 to 10 µM, or 3 to 10 µM, or 4 to 10 µM, or 5 to 10 µM, or 6 to 10 µM, or 7 to 10 µM, or 8 to 10 µM, or 9 to 10 µM, preferablyl 3 µM, 5 µM, 8 µM, or 10 µM.

### XII. cMet Inhibitor

Receptor tyrosine kinase (cMet) is a receptor of hepatocyte growth factor (HGF). HGF/c-Met signal pathway is frequently activated during tumor formation, growth and metastasis. C-Met inhibitor is a small molecular compound, including, but not limited to, Cabozantinib (an effective VEGFR2 inhibitor), Capmatinib (a novel competitive ATP c-MET inhibitor). cMet inhibitor used in the culture system as described herein is preferably Capmatinib with the following structure:

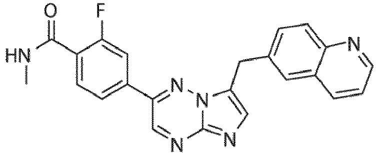

In the culture system for chemical induction of pluripotent stem cells as described herein, Capmatinib has a concentration from 0.01 to 10 µM, or 0.1 to 10 µM, or 1 to 10 µM, or 2 to 10 µM, or 3 to 10 µM, or 4 to 10 µM, or 5 to 10 µM, or 6 to 10 µM, or 7 to 10 µM, or 8 to 10 µM, or 9 to 10 µM, preferablyl 3 µM, 5 µM, 8 µM, or 10 µM.

### XIII. Vitamin C

Vitamin C has a similar structure to glucose, which is a compound with multiple hydroxyl groups, in which two enolic hydroxyl groups adjacent to each other on the second and the third positions are easily dissociated to release H+, thereby exhibiting acidity. Therefore, vitamin C is also known as ascorbic acid with the following structure:

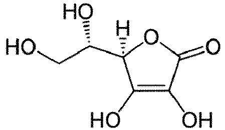

In the culture system for chemical induction of pluripotent stem cells as described herein, vitamin C has a concentration from 0.01 to 50 µg/ml, or 0.1 to 50 µg/ml, or 1 to 50 µg/ml, or 5 to 50 µg/ml, or 10 to 50 µg/ml, or 20 to 50 µg/ml, or 30 to 50 µg/ml, or 40 to 50 µg/ml, preferably 5 µg/ml, 10 µg/ml, 20 µg/ml, 30 µg/ml, 40 µg/ml or 50 µg/ml.

In one embodiment, the culture system for chemical induction of pluripotent stem cells comprises a basic medium such as iCD1 and a composition for performing chemical induction of reprogramming process comprising:
10ng/ml BMP4,
50µg/ml vitamin C,
5µM EPZ5676,
10µM Brdu,
0.05µM AM580,
3 µM CHIR99021,
10ng/ml FGF2,
10µM Forsklin,
5µM RepSox,
0.1 mM valproic acid,
0.05µM DZNep,
5µM SGC0946.

In another embodiment, the culture system for chemical induction of pluripotent stem cells comprises a basic medium such as iCD1 and a composition for performing chemical induction of reprogramming process comprising:
10ng/ml BMP4,
50µg/ml vitamin C,
5µM EPZ5676,
10µM Brdu,
0.05µM AM580,
3 µM CHIR99021,
10ng/ml FGF2,
10µM Forsklin,
5µM RepSox,
0.1 mM valproic acid,
0.05µM DZNep,
5µM SGC0946,
5µM EPZ6438,
5µM Capmatinib.

During performing chemical induction of reprogramming process by using the culture system as provided herein, there is no need to frequencely replate cells or use serum. Therefore, the reprogramming process is simplified, and loss of cells due to replating is reduced. And since the culture system as decribed herein is free of serum, the subsequent collection of pluripotent stem cells and molecular mechanism analysis will be simplified, thereby facilitating to establish no animal origin culture systems for induction of pluripotent stem cells.

### Methods for chemically inducing somatic cells to perform reprogramming process

In another aspect of this embodiment, provided herein is a method for chemically inducing somatic cells to perform reprogramming process, comprising culturing the somatic cells in the culture system as described herein for a time period that is sufficient to reprogram the somatic cells to pluripotent state. The method further comprises changing the culture medium to a second medium supplement with PD0325901, non-essential amino acids, GlutaMax™, human white cell antigen B27, CHIR99021, leukaemia inhibitory factor, and/or N2 additive after the somatic cells have been reprogrammed to pluripotent state and culturing the reprogrammed cells for a second time period that is sufficient to maintain the native state of the embryonic stem cells.

In one embodiment, the second medium comprises 1 µM PD0325901, 1% non-essential amino acid of the total volume of the medium, 1% GlutaMax™ of the total volume of the medium, 2% human white cell antigen B27 of the total volume of the medium, 3 µM CHIR99021, 1% N2 additive of the total volume of the medium and 1U to 1000U leukaemia inhibitory factor.

In one embodiment, the method for inducing somatic cells to perform chemical induction of reprogramming process comprises culturing the somatic cells in the culture system as provided herein for 8 days to 22 days, preferably 22 days, and then changing the medium to the second medium as mentioned above and continuing to culture for 1 day to 18 days, preferably 18 days.

### Kit

In yet another aspect of this embodiment, provided herein is a kit for chemical induction of somatic cells to perform reprogramming process, comprising the composition for performing chemical induction of reprogramming process as mentioned above and a basic medium, and optionally an instruction for the culture system, a culture plate for culturing cells. The kit may further comprise tools for collecting cell or tissue samples from donors, a culture flask for the preservation of the cell or tissue samples, etc. The instruction included in the kit can provide users with uses of the composition and related information. The instruction can be of any suitable forms, including, but not limited to, publications, videos, computer readable discs or compact discs.

### EXAMPLES

Mouse embryo fibroblasts, mouse neonatal fibroblasts, mouse lung fibroblasts, mouse tail tip fibroblasts, mouse neural stem cells and mouse hepatocytes were selected as examples to illustrate the culture system for chemical induction of pluripotent stem cells, as provided herein. Mouse embryo fibroblasts used in this example were from OG2 mouse E13.5 embryo. Mouse neonatal fibroblasts were from derm layer of OG2 mouse 1 to 3 days after mouse was born. Mouse tail tip fibroblasts were from tail connective tissues of 6 to 8-week old OG2 mouse. Mouse lung fibroblasts were from lung tissues of 6 to 8-week old OG2 mouse. These fibroblasts were maintained in DMEM medium supplement with 10% FBS, GlutMax (100x) and NEAA (100x). The mouse neural stem cells used in this example were from brain tissue of OG2 mouse E13.5 embryo and maintained in DMEM/F12 medium supplement with 1% N2, 2% B27, bFGF (10ng/ml), EGF (10ng/ml), GlutMax (100x) and NEAA (100x). Mouse stem cells used as control were from liver tissue of 6 to 8-week old OG2 mouse and maintained in HCM medium.

### Example 1: Production of pluripotent stem cells in the culture system for chemical induction of pluripotent stem cells

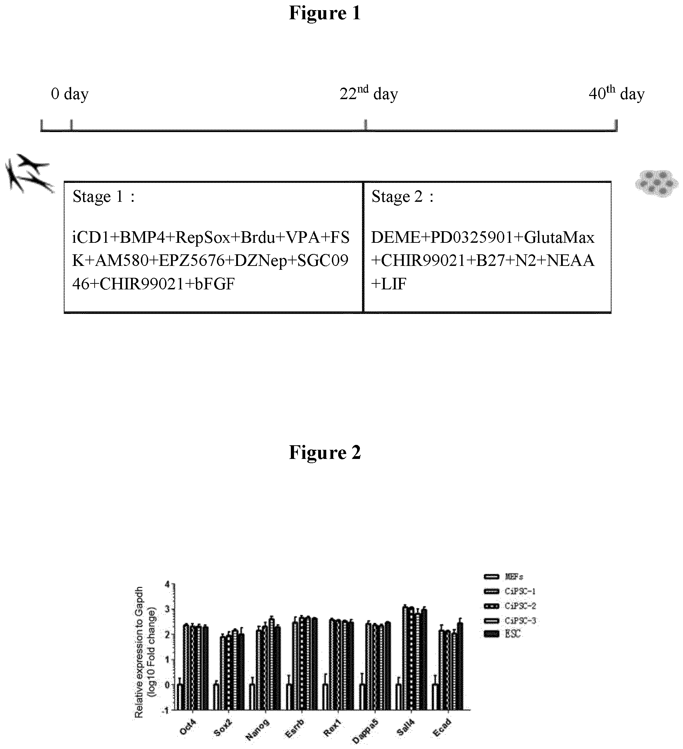

The culture medium for use in Example 1 compises iCD1 medium as a basic medium and 10ng/ml BMP4, 10µM Brdu, 5µM RepSox, 10µM Forsklin, 0.1 mM VPA, 0.05µM AM580, 5µM EPZ5676, 0.05µM DZNep, 5µM SGC0946, 50µg/ml vitamin C, 3µM CHIR99021 and 10ng/ml basic fibroblast growth factor. Mouse embryo fibroblasts, mouse neonatal fibroblasts, mouse lung fibroblasts, mouse tail tip fibroblasts, mouse neural stem cells were seeded in the medium for chemical induction of pluripotent stem cells at a density of 20,000 cells per well (12 wells) or 50,000 cells per well (6 wells) and the mouse hepatocytes were seeded in the same medium for chemical induction of pluripotent stem cells at a density of 500,000 cells per well (6-well plate). The medium was freshed on daily basis. After culturing for 22 days, the above medium for chemical induction of pluripotent stem cells was changed to DMEM medium comprising N2 (100X), B27 (50X), GlutMax (100X), NEAA (100X), 3 µM CHIR99021, 1 µM PD0325901 and LIF (1000U) and then continued culturing for 14 days to 18 days (as shown in Figure 1). The chemically induced pluripotent stem cells (ciPSC) were collected for analysis.

### Example 2. Characterization of the chemically induced pluripotent stem cells (ciPSCs)

Expression levels of endogenous pluripotent genes Oct4, Nanog, Sox2, Esrb, Rex1Dappa5, Sall4 and Cdhl in ciPSCs were measured by quantitative RT-PCR. As shown in Figure 2, the expression levels of these endogenous pluripotent genes were the same as those measured in the mouse embryo stem cells.

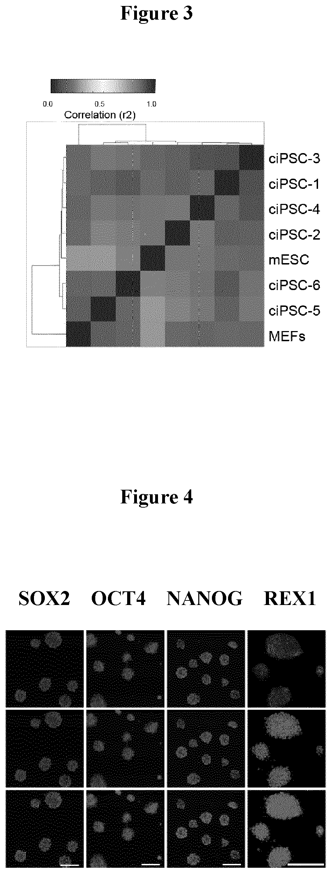

Transcriptomic profiles of ciPSCs were obtained via RNA-seq. As shown in Figure 3, the transcriptomic profiles of ciPSCs were the same as that of the mouse embryo stem cells.

Further, as shown in Figure 4, the protein expression levels of endogenous pluripotent genes Oct4, Nanog, Sox2, Esrb, Rex1Dappa5, Sall4 and Cdhl in ciPSCs were confirmed by immunofluorescence.

### Example 3. Differentiation profile of chemically induced pluripotent stem cells in mouse

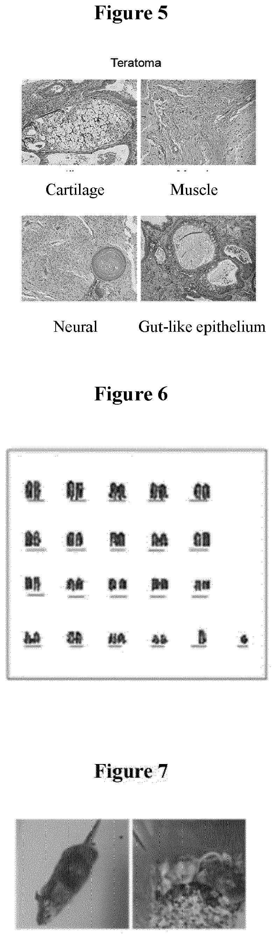

ciPSCs obtained according to example 1 were injected to NOD-SCID mouse. As shown in Figure 5, ciPSCs formed teratoma and were differentiated into three germ layers (cartilage: mesoderm, muscle: mesoderm, nerve: ectoderm; gut-like epithelium: entoderm) in mouse. As shown in Figure 6, ciPSCs were maintained with normal karyotype during passage. ciPSCs were injected into mouse blastocyst and then progeny chimeric mouse was obtained, as shown in Figure 7.

As demonstrated in the results of Examples 2 and 3, the chemically induced pluripotent stem cells obtained by culturing in the culture system as described herein have complete pluripotency. By using the culture system as described herein, somatic cells can be effectively reprogrammed into pluripotent stem cells through two-stage culturing process without any serum. And there is no need to replate cells during culture, such that the culturing process is simplified. As the culture system is free of serum, subsequent collection of pluripotent stem cells and molecular mechanism analysis are simplified, thereby facilitating establishment of no animal origin culture systems for induction of pluripotent stem cells.

### Test Method

Quantitative RT-PCR analysis and RNA-seq analysis. Total RNAs were isolated from cells with TRIzol and converted into cDNAs with ReverTra Ace (Toyobo) and oligo-dT (Takara), and then analysed by qPCR with Premix Ex Taq (Takara). TruSeq RNA Sample Prep Kit (RS-122-2011, Illumina) was used for library constructions and sequencing was done with Miseq Reagent Kit V2 (MS-102-2001, Illumina) for RNA-seq. The primers used in this project can be found in Table 1.

Immunofluroscence staining. The cells were cultured in coverlips and fixed with 4% paraformaldehyde for 30 min at room temperature. Then the cells were washed with PBS for three times and permeated with 0.1% Triton X-100 for 30 min. Afterwards, the cells were blocked by 3% BSA in PBS for 1 hour at room temperature and incubated with primary antibodies at 4°C overnight. Next day, the cells were washed with PBS for three times and incubated with appreciated secondary antibodies for 1 hour. Besides, the nucleus were stained with DAPI. Finally, the coverlips were mounted on the slide for observation under the confocal microscope (Zeiss 710 NLO). Primary and secondary antibodies were anti-Oct4 (SC-5279, 1:400), anti-Sox2 (sc-17320, 1:200), anti-Nanog (BETHYL no. A300-397A, 1:200), anti-Rexl (SC-50668, 1:100) and anti-SSEAl (RD, MAB2155, 1:100) and diluted in 3% BSA.

Teratoma formation and generation of chimeric mouse. 1 x 10⁶ ciPSCs were subcutaneously injected into NOD SCID mouse and teratoma was formed from 4 to 8 weeks. For generation of chimeric mouse, ciPSCs were injected into ICR blastocysts which were transplanted into pseudopregnant ICR female mouse. The resulting chimeric mouse was used for germline transmission by mating F2 mouse with ICR mouse.

**Table 1. Primers used in sequencing.**

| **Genes** | **Sense sequence (5' to 3')** | **Anti-sense sequence (5' to 3')** |
|---|---|---|
| Oct4 | CATTGAGAACCGTGTGAG (SEQ ID NO.:1) | TGAGTGATCTGCTGTAGG (SEQ ID NO.:2) |
| Nanog | CTCAAGTCCTGAGGCTGACA (SEQ ID NO.:3) | TGAAACCTGTCCTTGAGTGC (SEQ ID NO.:4) |
| Sox2 | AGGGCTGGGAGAAAGAAGAG (SEQ ID NO.:5) | CCGCGATTGTTGTGATTAGT (SEQ ID NO.:6) |
| Esrrb | TTTCTGGAACCCATGGAGAG (SEQ ID NO.:7) | AGCCAGCACCTCCTTCTACA (SEQ ID NO.:8) |
| Dppa5 | CCGTGCGTGGTGGATAAG (SEQ ID NO.:9) | GCGACTGGACCTGGAATAC (SEQ ID NO.: 10) |
| Rex1 | CAGCCAGACCACCATCTGTC (SEQ ID NO.:11) | GTCTCCGATTTGCATATCTCCTG (SEQ ID NO.:12) |
| Sall4 | CTAAGGAGGAAGAGGAGAG (SEQ ID NO.:13) | CAAGGCTATGGTCACAAG (SEQ ID NO.: 14) |
| Sox17 | CAGTATCTGCCCTTTGTGTA (SEQ ID NO.: 15) | GCAATAGTAGACCGCTGAG (SEQ ID NO.: 16) |
| Foxa2 | GCAGACACTTCCTACTACC (SEQ ID NO.:17) | TCCACTCAGCCTCTCATT (SEQ ID NO.:18) |
| Gata4 | CAGCAGCAGTGAAGAGAT (SEQ ID NO.:19) | GTCTGAGTGACAGGAGATG (SEQ ID NO.:20) |
| Gata6 | GGTCTCTACAGCAAGATGAATGG (SEQ ID NO.:21) | TGGCACAGGACAGTCCAAG (SEQ ID NO.:22) |
| Cdh1 | CAGCCTTCTTTTCGGAAGACT (SEQ ID NO.:23) | GGTAGACAGCTCCCTATGACTG (SEQ ID NO.:24) |
| Epcam | CTTGTGTCTGCACGACCTGT (SEQ ID NO.:25) | CCAAGCATTTAGACGCCAGTTT (SEQ ID NO.:26) |
| Cdh2 | CCATCATCGCTATCCTTCT (SEQ ID NO.:27) | CCTCCACCTTCTTCATCATA(SEQ ID NO.:28) |
| CD44 | CGTTAATGTTGATGGCTCCTTAC (SEQ ID NO.:29) | GTCCTGGTTCGCACTTGA (SEQ ID NO.:30) |
| Twist2 | AGATGACCAGCTGCAGCTAC (SEQ ID NO.:31) | ATGTGCAGGTGGGTCCTG (SEQ ID NO.:32) |
| Snail | CTCGGATGTGAAGAGATACC (SEQ ID NO.:33) | AGACTCTTGGTGCTTGTG (SEQ ID NO.:34) |
| Gapdh | AACTTTGGCATTGTGGAAGGGCT CA (SEQ ID NO.:35) | TTGGCAGCACCAGTGGATGCAGGG A (SEQ ID NO.:36) |

Although the preferred embodiments of the present invention have been described and illustrated herein, it is obvious to those skilled in the art that these embodiments are only for the purpose of illustration. It will be apparent to those skilled in the art that numerous variations, modifications and substitutions can be made to these embodiments without departing from the scope and spirit of the present invention. The scope of the present invention is defined by the appended claims and the methods and structures as fall within the claims together with the equivalents thereof are intended to be embraced by the appended claims.

## Claims

1. A culture system for chemical induction of pluripotent stem cells, comprising a medium and a group consisting of: a thymine analogue, a cAMP activator, a TGF-β receptor inhibitor, a bone morphogenetic protein, a RA receptor activator, a GSK3 inhibitor and a basic fibroblast growth factor, wherein the said culture system is free of serum.

2. The culture system of claim 1, wherein,
the thymine analogue has a concentration from 0.01µM to 10 µM,
the cAMP activator has a concentration from 0.01 µM to 10 µM,
the TGF-β receptor inhibitor has a concentration from 0.01 µM to 5 µM,
the bone morphogenetic protein has a concentration from 0.01 ng/ml to 10 ng/ml,
the basic fibroblast growth factor has a concentration from 0.01 ng/ml to 10 ng/ml,
the RA receptor activator has a concentration from 0.01 µM to 0.05 µM, and/or
the GSK3 inhibitor has a concentration from 0.01 µM to 3 µM.

3. The culture system of claim 2, wherein,
the thymine analogue is Brdu,
the cAMP activator is FSK,
the TGF-β receptor inhibitor is RepSox,
the bone morphogenetic protein is BMP4,
the basic fibroblast growth factor is FGF2,
the RA receptor activator is AM580, and/or
the GSK3 inhibitor is CHIR99021.

4. The culture system of any one of claims 1 to 3, further comprising vitamin C, DOT1L inhibitor, histone deacetylase inhibitor and/or DZNep.

5. The culture system of claim 4, wherein,
the vitamin C has a concentration from 0.01 µg/ml to 50 µg/ml,
the DOT1L inhibitor has a concentration from 0.01 µM to 5 µM,
the histone deacetylase inhibitor has a concentration from 0.01 mM to 0.5 mM,
the DZNep has a concentration from 0.001 µM to 0.05 µM.

6. The culture system of claim 5, wherein,
the DOT1L inhibitor is EPZ5676 and/or SGC0946,
the histone deacetylase inhibitor is valproic acid.

7. The culture system of any one of claims 1 to 3, further comprising EZH2 inhibitor and/or cMet inhibitor.

8. The culture system of claim 7, wherein, the EZH2 inhibitor has a concentration from 0.01 to 10 µM, and/or the cMet inhibitor has a concentration from 0.01 to 10 µM.

9. The culture system of claim 8, wherein, the EZH2 inhibitor is EPZ6438, and the cMet inhibitor is Capmatinib.

10. The culture system of claim 6, comprising:
10ng/ml BMP4 ;
50µg/ml vatimin C ;
5µM EPZ5676 ;
10µM Brdu ;
0.05µM AM580 ;
3 µM CHIR99021 ;
10ng/ml FGF2 ;
10µM Forsklin ;
5µM RepSox ;
0.1 mM valproic acid ;
0.05µM DZNep ; and/or
5µM SGC0946.

11. The culture system of claim 9, comprising:
10ng/ml BMP4 ;
50µg/ml vatimin C ;
5µM EPZ5676 ;
10µM Brdu ;
0.05µM AM580 ;
3 µM CHIR99021 ;
10ng/ml FGF2 ;
10µM Forsklin ;
5µM RepSox ;
0.1 mM valproic acid ;
0.05µM DZNep ;
5µM SGC0946 ;
5µM EPZ6438 ; and/or
5µM Capmatinib.

12. The culture system of any one of claims 1 to 11, wherein, the medium is iCD1 medium.

13. A method for chemical induction of somatic cells to perform reprogramming process, comprising culturing the somatic cells in the culture system of any one of claims 1 to 12 for a time period that is sufficient to reprogram the somatic cells to pluripotent state.

14. The method of claim 13, further comprising changing the medium to DMEM medium supplement with PD0325901, non-essential amino acids, GlutaMax™, human white cell antigen B27, CHIR99021, leukaemia inhibitory factor, and/or N2 additive after the somatic cells have been reprogrammed to pluripotent state and culturing the reprogrammed cells for a second time period that is sufficient to maintain the native state of the embryonic stem cells.

15. The method of claim 14, wherein,
PD0325901 has a concentration of 1 µM,
the non-essential amino acids are comprised of 1% of the total volume of the medium,
GlutaMax™ is comprised of 1% of the total volume of the medium,
the human white cell antigen B27 is comprised of 2% of the total volume of the medium,
CHIR99021 has a concentration of 3 µM,
the N2 additive is comprised of 1% of the total volume of the medium, and/or
the leukaemia inhibitory factor has a concentration from 1U to 1000U.

16. The method of claim 13, wherein, the time period that is sufficient to reprogram the somatic cells to pluripotent state is 8 days to 22 days.

17. The method of claim 14, wherein, the second time period that is sufficient to maintain the native state of the embryonic stem cells is 1 day to 18 days.

18. The method of any one of claims 9 to 13, wherein, the somatic cells are selected from the group consisting of fibroblasts, bone-marrow derived monocytes, skeletal muscle cells, adipocytes, peripheral blood monouclear cells, macrophages, hepatocytes, keratinocytes, oral keratinocytes, hair follicle dermal cells, stomach epithelial cells, lung epithelial cells, synoviocytes, renal cells, skin epithelial cells, osteoblasts, neural stem cells and dermal cells.

19. A kit for chemical induction of pluripotent stem cells, comprising the culture system of any one of claims 1 to 12.
